# EUROPEAN PATENT APPLICATION

(11) **EP 3 513 716 A1**
(43) Date of publication of application: **24.07.2019**
(21) Application number: 18152538.7
(22) Date of filing: 19.01.2018
(51) Int. Cl.: A61B 5/021, A61B 5/0488, A61B 5/11, A61B 5/22, A61B 5/00

(54) **A SYSTEM AND METHOD FOR MONITORING HEART STRAIN OF A SUBJECT**

(71) Applicant: Koninklijke Philips N.V., 5656 AE Eindhoven (NL)
(72) Inventor: SARTOR, Francesco, 5656 AE Eindhoven (NL); PAPINI, Gabriele, 5656 AE Eindhoven (NL); DE MORREE, Helma Majella, 5656 AE Eindhoven (NL)
(74) Representative: de Haan, Poul Erik

(57) **Abstract**

A system and method is provided for monitoring heart strain of a subject, in which an effort sensor signal is used to determine when a blood pressure has exceeded a blood pressure threshold and then provide a warning. The correlations between the exerted effort and a lifted weight, and between the lifted weight and the (systolic) blood pressure are used to provide a blood pressure warning, in particular for patients with a heart condition or post-heart treatment.

## Description

### FIELD OF THE INVENTION

This invention relates to a system and method for monitoring heart strain of a subject, in particular for monitoring subjects after undergoing a medical procedure relating to their heart or having a medical heart condition.

### BACKGROUND OF THE INVENTION

Lifting, or more in general terms displacing, a weight puts a strain on the heart. This applies to lifting of any type of weight, such as a chair or a box at home or at work, to a dumbbell at the gym. Weight lifting increases the pressure that the left ventricle of the heart needs in order to push the blood out into the main stream circulation. This pressure is referred to as the afterload. The systolic blood pressure (SBP) is directly related to this afterload.

An increased afterload in a weakened heart, for instance following a heart attack, can result in valve regurgitation (a leaking valve) and this can lead to heart failure.

For this reason, heart patients are told to avoid lifting heavy weights or performing unnecessary effort which could put their heart under strain. However, patients do not know what level of physical effort is dangerous for their heart.

There are two possible outcomes which can result. One is that the person takes no risk and instead limits their normal life activity substantially, which means they may become far more dependent than they could safely be. The other is that the person takes undue risk by carrying on with activities they were able to perform before the heart event. This carries the risk of further damage to the heart.

It is known to provide monitoring of physiological parameters during exercise, for example to identify the start, end and level of exercise. WO 2016/157217 for example discloses a system for assisting a user in performing a healthy exercise routine, for example to reduce the need for a personal trainer. It includes a sensor for measuring a force applied and grip pattern and a motion sensor. Different grip patterns correlate to different exercises. The system is implemented as a glove so that a contact force can be measured.

There is a need for a subject with a heart condition or following heart surgery to be able to monitor their activity levels, in particular when lifting objects, so that they can determine whether activities are safe having regard to their particular medical condition.

### SUMMARY OF THE INVENTION

The invention is defined by the claims.

According to examples in accordance with an aspect of the invention, there is provided a system for monitoring heart strain of a subject, comprising:
a sensor for deriving an effort applied by a subject to displace an object; and
a processor for processing the sensor output,
wherein the processor is adapted to:
   analyze the sensor output thereby to determine when a blood pressure has exceeded a blood pressure threshold; and
   provide a warning when the blood pressure is determined to have exceeded the blood pressure threshold.

This system provides a mapping between an effort applied by a subject to displace an object and a corresponding blood pressure level. It is based on the recognition that lifting or other movement of different weights correlates to different required effort, and that lifting of different weights also correlates to different blood pressure levels.

The effort may be a representation of a degree of force applied. However, it may involve an indirect measurement and hence not a direct measurement of a force associated directly with the load. For example, increased hand, forearm and arm activity levels (in the muscles and tendons) may represent a greater lifting force. This greater lifting force results in additional activity of the fingers as a result of the weight of the object being lifted, or otherwise displaced, and it may also correlate to a tightened grip to maintain a hold of the object.

The sensor is preferably a physiological sensor for measuring muscle or tendon condition changes. In this way, the sensor does not need to interact with the object being displaced but provides an indirect measurement of the nature of the object and its movement by means of the physiological effect on the user's muscles and tendons.

The effort is for example related to the external load being supported by the hand of the subject. The level of grip force applied by the user is a measure of the exertion of the subject, in particular their muscular exertion and this can be measured by physiological sensors. For example, the hand is heavily controlled by muscles and tendons located in the forearm and the arm. By monitoring either their movements, and/or electrical activity, and/or sound, and/or vibration, and/or perfusion, it is possible to derive the grip and/or force and/or pressure that the user applies to displace an object, and its intrinsic weight.

Thus, monitoring of hand, forearm, or arm physiological signals can be used to determine whether blood pressure levels are within a safe range, for a particular subject being monitored. In this way, blood pressure levels can be monitored in a simple manner requiring only a sensor arrangement which detects movement or forces. This can give minimal discomfort or inconvenience to the user of the system and also enables the sensing to be performed while the subject is moving. The system is able to provide real-time feedback to a subject about the load they can safely lift or otherwise displace according to their personal threshold.

In a most simple implementation, the processor provides an effort threshold which, for the particular subject, corresponds to a blood pressure threshold. Thus, the analysis of the sensor output may comprise comparison with a threshold, thereby to determine that the blood pressure has exceeded the blood pressure threshold.

In another example, the processor is adapted to:
convert the sensor output to a blood pressure estimate; and
compare the blood pressure estimate with the blood pressure threshold.

In this case, there is the intermediate calculation of a blood pressure value, which may for example be provided as an output of the system to provide additional information to the user. The blood pressure estimate is for example an estimate of systolic blood pressure.

The processor may be adapted to:
convert the sensor output to an estimate of a lifted weight; and
convert the estimate of a lifted weight to the blood pressure estimate.

In this case, there is the intermediate calculation of a lifted weight value, which may also be provided as an output of the system to provide additional information to the user.

The sensor is for example implemented as a wearable bracelet, armband or glove. This provides an easy to use wearable solution. The sensor may for example comprise a set of one or more electromyography sensors. These measure the muscle response. Instead, or in addition, sensors may be used to measure an external force applied.

The system may further comprise a motion sensor for sensing hand or arm motion. Information about the hand or arm motion can also be used to identify lifting actions and distinguish them from other displacement actions which have a different (and lesser) effect on blood pressure such as opening a jar.

The processor is for example adapted to implement a calibration routine, which comprises:
collecting effort measurements for a plurality of lifted weights;
collecting blood pressure measurements for the plurality of lifted weights; and
determining a threshold to be applied when analyzing the sensor output.

This calibration routine enables the warning to be tailored to the specific subject, by monitoring their physiological response to lifting known weights.

The invention also provides a method of monitoring heart strain of a subject, comprising:
obtaining a measure of effort while a subject displaces a weight;
analyzing the effort thereby to determine when a blood pressure has exceeded a blood pressure threshold; and
providing a warning when the blood pressure is determined to have exceeded the blood pressure threshold.

This method makes use of a correlation between effort applied and the weight of an object being lifted in order to provide a warning to a subject that a weight being lifted may result in a dangerously high blood pressure, having regard to the particular condition of the subject.

The method may comprise:
converting the effort measurement to a blood pressure estimate; and
comparing the blood pressure estimate with the blood pressure threshold.

This intermediate calculation enables a blood pressure estimate to be provided to the user. The blood pressure estimate is for example an estimate of systolic blood pressure.

The method may comprise:
converting the effort measurement to an estimate of a lifted weight; and
converting the estimate of a lifted weight to the blood pressure estimate.

This intermediate calculation enables a lifted weight estimation to be provided to the user.

The measure of effort is for example made using a wearable bracelet, armband or glove for example with a set of one or more electromyography sensors.

The method may further comprise sensing hand or arm motion and detecting a lifting action from the sensed hand or arm motion. In this way, actions which do not correspond to lifting a weight can be filtered from the results.

The method may comprise implementing a calibration routine, which comprises:
collecting effort measurements for a plurality of lifted weights;
collecting blood pressure measurements for the plurality of lifted weights; and
determining a threshold to be applied when analyzing the effort measurement.

This enables the warning to be tailored to a particular subject.

The invention may be implemented at least in part in software.

These and other aspects of the invention will be apparent from and elucidated with reference to the embodiment(s) described hereinafter.

### BRIEF DESCRIPTION OF THE DRAWINGS

Examples of the invention will now be described in detail with reference to the accompanying drawings, in which:
Figure 1 shows the relationship between a weight being lifted and the muscle activity and the relationship between the weight being lifted and the systolic blood pressure;
Figure 2 shows the corresponding relationship between finger flexor gripping force, EMG, and the systolic blood pressure for two different muscle groups;
Figure 3 shows a system for monitoring heart strain of a subject;
Figure 4 shows a method of monitoring heart strain of a subject; and
Figure 5 shows a generic computer for implementing the method of Figure 4.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for monitoring heart strain of a subject, in which an effort sensor signal is used to determine when a blood pressure has exceeded a blood pressure threshold and then provide a warning. The correlations between an effort level and a lifted weight, and between a lifted weight and the (systolic) blood pressure are used to provide a blood pressure warning, in particular for patients with a heart condition or post heart treatment.

The system and method indirectly evaluates the strain that the effort of lifting or displacing a weight puts on the heart (i.e. the afterload) any time the user lifts or displaces a weight. The warning is tailored to the particular diagnosis of the subject.

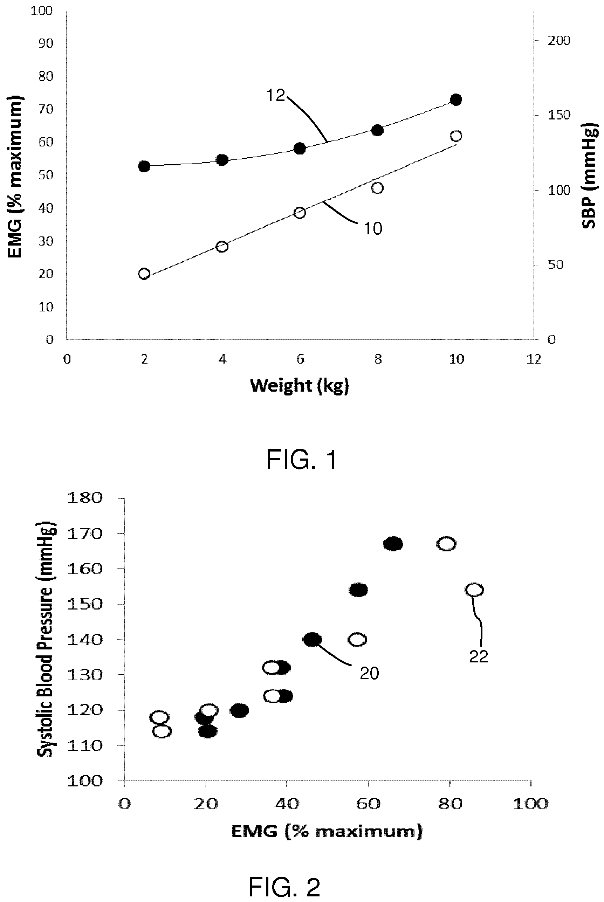

Figure 1 shows the relationship between a weight being lifted (x-axis) and the muscle activity (shown as an electromyography signal, EMG, using the left y-axis) and the relationship between the weight being lifted and the systolic blood pressure, SBP (right y-axis). Plot 10 represents the muscle activity measured via electromyography of a finger flexor muscle and plot 12 represents the blood pressure reading. Both plots show a strong positive correlation with the weight being lifted.

Figure 2 shows the corresponding relationship between the electromyography muscle activity, EMG, (which is representative of an effort level) and the systolic blood pressure for two different muscle groups (the upper arm biceps brachii muscle as plot 20, and the forearm brachioradialis and carpi radialis longus muscles as plot 22).

This shows that different muscle groups may be used as they show a near linear relationship between systolic blood pressure and muscle response.

Different implementations will make use of different physiological signal measurements. For example, for a sensor implemented as a watch, monitoring can be done by sensors located around the strap, which transduce tendon tension, vibration and movement. An EMG sensor and microphone can also be used, to transduce pronator quadratus, extensor indicis, extensor pollicis lungus, extensor pollicis brevis, and/or extensors tendon electrical activation or movements, respectively.

The sensor could also applied to the volar part of the wrist allowing the monitoring also of the flexors.

A glove is able to monitor muscle activity and tendon movements happening at the hand level. This is however limited to interossei muscles and finger tendons.

Another possible implementation is an armband. The forearm muscles such as brachioradialis and carpi radialis longus muscles can then be monitored. Monitoring of the arm may additionally include monitoring of the biceps brachii muscle.

Thus, the sensor may be for monitoring muscle or tendon movements or tension or electrical activity.

One preferred implementation is a wearable band to be located on the upper part of the forearm.

The muscle activity may that is monitored may represent tightness of the grip of the hand or the lifting effort for example in the muscles of the forearm in supporting the weight. Thus, there are different physiological signals that may be monitored. Direct monitoring of the force applied to the palm of the hand (i.e. to the palm of a sensing glove) may provide a more direct measurement of the load acting on the hand. However, this is preferred as an additional measure to the more indirect physiological measurements.

Different signals may be combined as part of the algorithm for providing a best representation of the effort being exerted by the subject.

Using the relationships between the weight being lifted and the effort level (manifested as a grip force (i.e. strain) and/or pulling force, or some combination of the two) and the between the effort level and systolic blood pressure, it becomes possible to recognize events for which the blood pressure, derived from the effort sensor signal analysis, corresponds to the blood pressure response which exceeds an acceptable blood pressure level set by a physician as index of permitted afterload.

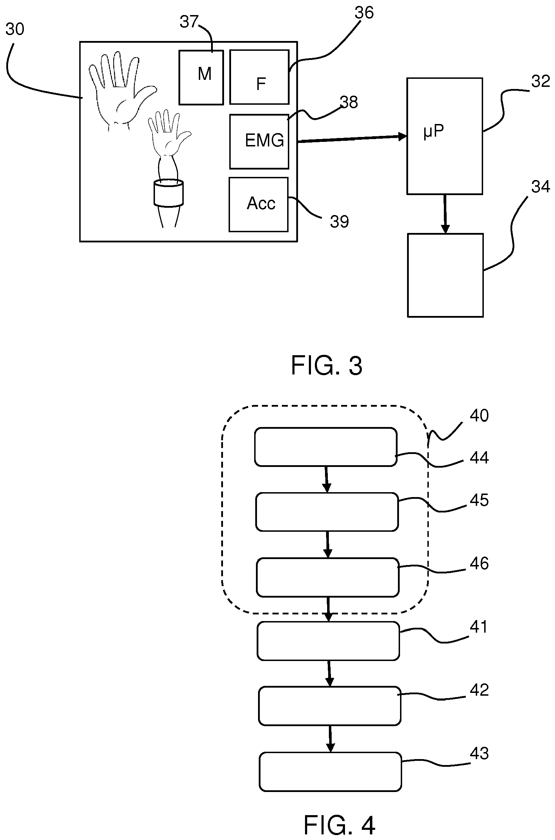

Figure 3 shows a system for monitoring heart strain of a subject. The system comprises an effort sensor 30 and a processor 32 for processing the effort sensor output. An output device 34 provides a warning to the user of the system when the user's blood pressure is determined to have exceeded a blood pressure threshold.

This determination is based on the relationships explained above. In particular, the effort sensor output is analyzed to determine when a blood pressure has exceeded the blood pressure threshold based on a mapping between the effort signal and a corresponding blood pressure level.

In a most simple implementation, the processor simply compares a measured effort level with a previously determined threshold which, for the particular subject, corresponds to a blood pressure threshold. Thus, there is no need for intermediate calculations, so that the actual blood pressure level and the actual weight being lifted do not need to be identified as such.

However, the processor may instead perform a first analysis in which the effort sensor output is converted to a blood pressure estimate and the blood pressure estimate is then compared with the blood pressure threshold. In this case, the output device 34 may also provide the blood pressure estimate as an output.

This approach basically involves using the function of Figure 2 to map between effort (the x-axis of Figure 2) and a blood pressure estimate (the y-axis of Figure 2) and a threshold level is set for the blood pressure.

The systolic blood pressure may be calculated, but any other generic indication of blood pressure may be provided. The blood pressure output may for example simply indicate a generic "normal", "slightly high", "very high" and "warning" for example, rather than a numeric indication of blood pressure level (which is unlikely to be very accurate).

The processor may perform a further analysis in which the effort sensor output is first converted to an estimate of a lifted weight before the conversion of the lifted weight to the blood pressure estimate. The lifted weight output may for example simply indicate a generic indication such as "OK", "heavy", "warning - too heavy" for example, rather than a numeric indication of a lifted weight (which is unlikely to be very accurate).

The effort sensor 30 for example comprises a wearable wrist band (e.g. smart watch) arm band or glove. There may be multiple sensors provided in the wearable sensor.

Figure 3 shows a force sensor 36, a microphone 37, an electromyography sensor 38 and a motion sensor 39, in particular an acceleration sensor.

The force sensor 36 may be used to measure an external pressure applied for example in a glove, to an object being held, for example based on a pressure sensor or strain sensor. This measures the pull force explained above, which is basically the load which is being supported by the hand. This is non-physiological measurement. The microphone can measure local movement for example of a tendon in order to derive tendon strain.

The electromyography sensor 38 measures muscle strain. The sensor comprises a surface EMG sensor, recording muscle activity from the surface above the muscle on the skin. A pair of surface electrodes may be used or there may be a more complex array of multiple electrodes. Other physiological measurements may be used.

The sensor is thus for measuring one or more of muscle and tendon movement, muscle electrical activity, muscle and tendon sound, muscle and tendon vibration, blood perfusion.

The motion sensor 39 measures global hand or arm movement. This can be used to identify lifting actions and thereby distinguish between lifting actions and other movements. This avoids other actions being identified as weight lifting actions.

As mentioned above, one preferred implementation is for a forearm band. In this case, the flexor carpi ulnaris, extensor carpi ulnaris, extensor digitorum, extensor carpi radialis brevis, extensor carpi radialis longus, brachioradialis, and the deeper muscles (as cross talk) like the supinator, abductor pollicis longus are monitored as far as the dorsal part is concerned. If the forearm band also has sensors in the volar part, the brachioradialis, pronator teres, flexor carpi radialis, palmaris longus, flexor carpi ulnari, and the deeper muscles (as cross talk) like the flexor digitorum superficialis and flexor digitorum profundis, and felxor pollics longus, maybe monitored as well.

These various muscle groups work usually as agonist/antagonist so in practice it is easier to measure all of them than a single muscle. On the forearm the solution would depend less on tendon movement. Tendon movement is more readily measured around the wrist.

Figure 4 shows a method of monitoring heart strain of a subject.

There is first a calibration stage, discussed below, during which a suitable threshold for a particular subject can be determined, and which is used to control the generation of a warning.

After calibration, the method comprises:
in step 41, obtaining a measure of effort while a subject lifts a weight;
in step 42, analyzing the effort measurement thereby to determine when a blood pressure has exceeded a blood pressure threshold; and
in step 43, providing a warning when the blood pressure is determined to have exceeded the blood pressure threshold.

As explained above, the analysis of the effort may comprise converting the effort measurement to a blood pressure estimate, and optionally previously converting the effort measurement to an estimate of a lifted weight.

The step 41 may further involve sensing hand or arm motion and detecting a lifting action from the sensed hand motion so that the effort analysis 42 only takes place for actions which have been identified as actions of lifting a weight.

The calibration routine 40 comprises:
in step 44, collecting effort measurements for a plurality of lifted weights;
in step 45, collecting blood pressure measurements for the plurality of lifted weights; and
in step 46, determining a threshold to be applied when analyzing the effort measurements.

The step 46 may be conducted by a physician who decides what level of lifted weight is safe for the particular subject. The blood pressure measurement may be performed using a separate sensor such as a blood monitoring cuff or a PPG sensor. This blood pressure monitoring may be carried out by a sensor which is external to the system and used by a physician only for the purposes of the calibration. However, the system may instead have the blood pressure monitoring system as part of the overall system for use in the calibration phase.

The system typically does not have direct blood pressure monitoring, primarily because current blood pressure monitoring cannot be achieved while performing free-living activities, for practical reasons, and for physiological hemodynamic reasons. For example having the arm position at a different level compared to the heart when in different postures or when in motion renders current blood pressure monitoring solutions inaccurate. While a PPG sensor maybe used to derive blood pressure measurement, such sensors also suffer from motion artefacts.

In one example, the processor makes use of a database to provide the required mapping between the measured effort and the blood pressure threshold. This database may be trained using general population data and/or using the calibration routine as described above. Thus, the calibration phase is not essential. Instead, a subject may be classified according to their age, weight, a strength assessment, their diagnosis, etc. and they can then be allocated to a suitable data set from the general training data.

The system described above makes use of a processor for processing data.

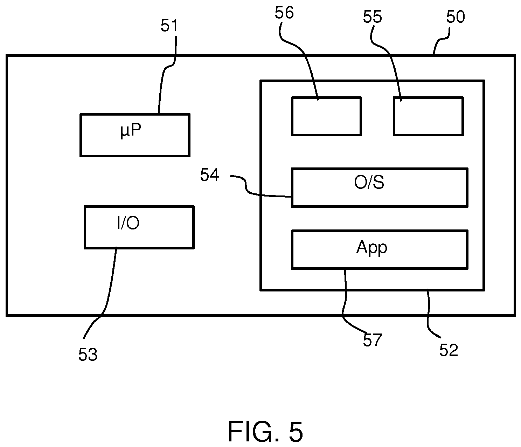

Figure 5 illustrates an example of a computer 50 for implementing the processor described above.

The computer 50 includes, but is not limited to, PCs, workstations, laptops, PDAs, palm devices, servers, storages, and the like. Generally, in terms of hardware architecture, the computer 50 may include one or more processors 51, memory 52, and one or more I/O devices 53 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 51 is a hardware device for executing software that can be stored in the memory 52. The processor 51 can be virtually any custom made or commercially available processor, a central processing unit (CPU), a digital signal processor (DSP), or an auxiliary processor among several processors associated with the computer 50, and the processor 51 may be a semiconductor based microprocessor (in the form of a microchip) or a microprocessor.

The memory 52 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 52 may incorporate electronic, magnetic, optical, and/or other types of storage media. Note that the memory 52 can have a distributed architecture, where various components are situated remote from one another, but can be accessed by the processor 51.

The software in the memory 52 may include one or more separate programs, each of which comprises an ordered listing of executable instructions for implementing logical functions. The software in the memory 52 includes a suitable operating system (O/S) 54, compiler 55, source code 56, and one or more applications 57 in accordance with exemplary embodiments.

The application 57 comprises numerous functional components such as computational units, logic, functional units, processes, operations, virtual entities, and/or modules.

The operating system 54 controls the execution of computer programs, and provides scheduling, input-output control, file and data management, memory management, and communication control and related services.

Application 57 maybe a source program, executable program (object code), script, or any other entity comprising a set of instructions to be performed. When a source program, then the program is usually translated via a compiler (such as the compiler 55), assembler, interpreter, or the like, which may or may not be included within the memory 52, so as to operate properly in connection with the operating system 54. Furthermore, the application 57 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 53 may include input devices such as, for example but not limited to, a mouse, keyboard, scanner, microphone, camera, etc. Furthermore, the I/O devices 53 may also include output devices, for example but not limited to a printer, display, etc. Finally, the I/O devices 53 may further include devices that communicate both inputs and outputs, for instance but not limited to, a network interface controller (NIC) or modulator/demodulator (for accessing remote devices, other files, devices, systems, or a network), a radio frequency (RF) or other transceiver, a telephonic interface, a bridge, a router, etc. The I/O devices 53 also include components for communicating over various networks, such as the Internet or intranet.

When the computer 50 is in operation, the processor 51 is configured to execute software stored within the memory 52, to communicate data to and from the memory 52, and to generally control operations of the computer 50 pursuant to the software. The application 57 and the operating system 54 are read, in whole or in part, by the processor 51, perhaps buffered within the processor 51, and then executed.

When the application 57 is implemented in software it should be noted that the application 57 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

## Claims

1. A system for monitoring heart strain of a subject, comprising:
a sensor (30) for deriving an effort applied by a subject to displace an object; and
a processor (32) for processing the sensor output,
wherein the processor is adapted to:
analyze the sensor output thereby to determine when a blood pressure has exceeded a blood pressure threshold; and
provide a warning when the blood pressure is determined to have exceeded the blood pressure threshold.

2. A system as claimed in claim 1, wherein the sensor (30) is adapted to provide a measurement of effort without direct contact with the object being displaced.

3. A system as claimed in claim 1 or 2, wherein the sensor (30) comprises a physiological sensor for measuring muscle or tendon activity.

4. A system as claimed in any preceding claim, wherein the processor (32) is adapted to:
convert the sensor output to a blood pressure estimate such as an estimate of systolic blood pressure; and
compare the blood pressure estimate with the blood pressure threshold.

5. A system as claimed in claim 2, 3 or 4, wherein the processor (32) is adapted to:
convert the sensor output to an estimate of a lifted weight; and
convert the estimate of a lifted weight to the blood pressure estimate.

6. A system as claimed in any preceding claim, wherein sensor (30) is implemented as a wearable bracelet, armband or glove for example comprising a set of one or more electromyography sensors.

7. A system as claimed in any preceding claim, wherein the sensor (30) further comprises a motion sensor (39) for sensing hand or arm motion.

8. A system as claimed in any preceding claim, wherein the processor is adapted to implement a calibration routine, which comprises:
collecting sensor measurements for a plurality of lifted weights;
collecting blood pressure measurements for the plurality of lifted weights; and
determining a threshold to be applied when analyzing the sensor output.

9. A method of monitoring heart strain of a subject, comprising:
(41) obtaining a measure of user effort while the user displaces a weight;
(42) analyzing the effort thereby to determine when a blood pressure has exceeded a blood pressure threshold; and
(43) providing a warning when the blood pressure is determined to have exceeded the blood pressure threshold.

10. A method as claimed in claim 9, comprising:
converting the measure of user effort to a blood pressure estimate such as an estimate of systolic blood pressure; and
comparing the blood pressure estimate with the blood pressure threshold.

11. A method as claimed in claim 9 or 10, comprising:
converting the measure of user effort to an estimate of a lifted weight; and
converting the estimate of a lifted weight to the blood pressure estimate.

12. A method as claimed in any one of claims 9 to 11, comprising obtaining the measure of user effort using a wearable bracelet, armband or glove, for example which comprises a set of one or more electromyography sensors.

13. A method as claimed in any one of claims 9 to 13, further comprising sensing hand or arm motion and detecting a lifting action from the sensed hand motion.

14. A method as claimed in any one of claim 9 to 13, comprising implementing a calibration routine (40), which comprises:
(44) collecting effort measurements for a plurality of lifted weights;
(45) collecting blood pressure measurements for the plurality of lifted weights; and
(46) determining a threshold to be applied when analyzing the effort measurement.

15. A computer program comprising computer program code means which is adapted, when said program is run on a computer, to implement the method of any one of claims 9 to 14.
